# EUROPÄISCHE PATENTSCHRIFT

(11) **EP 1 507 519 B1**
(45) Veröffentlichungstag und Bekanntmachung des Hinweises auf die Patenterteilung: **17.05.2006**
(21) Anmeldenummer: 03732464.7
(22) Anmeldetag: 26.05.2003
(51) Int. Cl.: A61K 31/13, A61K 9/20, A61K 9/16, A61P 25/00, A61P 13/00, A61P 29/00

(54) **1-DIMETHYLAMINO-3-(3-METHOXY-PHENYL)-2-METHYL-PENTAN-3-OL ENTHALTENDES ARZNEIMITTEL MIT VERZÖGERTER WIRKSTOFFFREISETZUNG**
MEDICAMENT WITH DELAYED RELEASE OF THE ACTIVE SUBSTANCE, CONTAINING 1-DIMETHYLAMINO-3-(3-METHOXY-PHENYL)-2-METHYL-PENTAN-3-OL
MEDICAMENT CONTENANT DU 1-DIMETHYLAMINO-3-(3-METHOXYPHENYL)-2-METHYLPENTAN-3-OL, LIBERANT LE PRINCIPE ACTIF DE MANIERE RETARDEE

(30) Priorität: 29.05.2002 DE 10224108
(43) Veröffentlichungstag der Anmeldung: 23.02.2005
(73) Patentinhaber: Grünenthal GmbH, 52078 Aachen (DE)
(72) Erfinder: BARTHOLOMÄUS, Johannes, 52080 Aachen (DE); ZIEGLER, Iris, 52159 Rott-Roetgen (DE)
(86) Internationale Anmeldenummer: PCT/EP2003/005488
(87) Internationale Veröffentlichungsnummer: WO 2003/099267

(56) Entgegenhaltungen:
- EP-A- 0 642 788
- EP-A- 0 693 475
- WO-A-02/43715
- WO-A-03/024444

## Beschreibung

Die Erfindung betrifft eine pharmazeutische Formulierung mit verzögerter Wirkstofffreisetzung, die 1-Dimethylamino-3-(3-methoxy-phenyl)-2-methyl-pentan-3-ol oder eines seiner pharmzeutisch akzeptablen Salze in einer Matrix enthält.

1-Dimethylamino-3-(3-methoxy-phenyl)-2-methyl-pentan-3-ol ist aus der EP 0 693 475 B1 als ein analgetisch wirksames Arzneimittel bekannt und kann oral appliziert werden. Die üblichen Formulierungen für die orale Verabreichung von 1-Dimethylamino-3-(3-methoxy-phenyl)-2-methyl-pentan-3-ol führen zu einer raschen Freisetzung des Wirkstoffs im Gastrointestinaltrakt, so daß seine analgetische Wirkung schnell einsetzt. Zugleich beobachtet man ein rasches Abklingen der Wirkung. Somit erfordert die Behandlung starker chronischer Schmerzen mit 1-Dimethylamino-3-(3-methoxy-phenyl)-2-methyl-pentan-3-ol bislang die Verabreichung des Arzneimittels in relativ kurzen Abständen, beispielsweise vier- bis sechsmal täglich, um so eine ausreichende Wirkstoffkonzentration im Blutplasma des Patienten zu gewährleisten. Die Notwendigkeit einer häufigen Dosierung führt jedoch leicht zu Fehlern bei der Einnahme sowie zu unerwünschten Plasmakonzentrationsschwankungen, was der Patientencompliance und dem therapeutischen Nutzen abträglich ist, insbesondere bei der Behandlung chronischer Schmerzzustände. Eine pharmazeutische Darreichungsform mit verzögerter Freisetzung (Retardformulierung) für die orale Applikation des Wirkstoffs 1-Dimethylamino-3-(3-methoxy-phenyl)-2-methyl-pentan-3-ol ist daher wünschenswert.

Im Stand der Technik sind allgemein Retardformulierungen für eine große Zahl verschiedener Wirkstoffe bekannt. Übliche Retardierungsformen sind u.a. Überzugsretardierungen und Matrixretardierungen.

Im Falle von Überzugsretardierungen, wie sie z.B. in der DE 36 25 458 A1 beschrieben sind, wird der einen Wirkstoff enthaltende Kern einer pharmazeutischen Zusammensetzung mit einem die Freisetzung des Wirkstoffs verzögernden Überzug aus einem oder mehreren hydrophilen und/oder hydrophoben Polymeren versehen.

Bei Matrixretardierungen ist der Wirkstoff in einer aus einem oder mehreren Trägermaterialien gebildeten Matrix enthalten, welche die Freisetzung des Wirkstoffs steuert. So offenbart beispielsweise die DE 33 09 516 A1 ein Verfahren zur Herstellung von Matrixformulierungen mit Hydroxypropylmethylcellulose (HPMC) als Trägermaterial und zum Teil verzögerter Freisetzung des Wirkstoffs, wobei das Trägermaterial nicht mehr als ein Drittel des Gewichts der Formulierung ausmacht und aus mindestens einer Hydroxypropylmethylcellulose besteht, die einen Methoxygehalt von 16-24 Gew.-%, einen Hydroxypropylgehalt von 4-32 Gew.-% und ein zahlenmäßig durchschnittliches Molekulargewicht von mindestens 50.000 aufweist. Die in der DE 33 09 516 A1 offenbarten Formulierungen enthalten HPMCs mit Viskositäten (in 2 gew.-%iger wäßriger Lösung bei 20 °C) zwischen 15 und 30.000 cPs (15 bis 30.000 mPa•s). Ein vom pH-Wert des Auflösungsmediums unabhängiges Freisetzungsverhalten wird in der DE 33 09 516 A1 nicht offenbart.

Es ist somit eine Aufgabe der vorliegenden Erfindung, eine 1-Dimethylamino-3-(3-methoxy-phenyl)-2-methyl-pentan-3-ol enthaltende pharmazeutische Formulierung mit verzögerter Wirkstofffreisetzung bereitzustellen.

Gelöst wird diese Aufgabe durch eine pharmazeutische Formulierung mit verzögerter Freisetzung, die 1-Dimethylamino-3-(3-methoxy-phenyl)-2-methyl-pentan-3-ol oder ein pharmazeutisch annehmbares Salz davon in einer Matrix mit verzögerter Wirkstofffreisetzung enthält,
wobei die Matrix 1 bis 80 Gew.-%, bevorzugt 5 bis 80 Gew.-%, eines oder mehrerer hydrophiler oder hydrophober Polymere als pharmazeutisch annehmbaren Matrixbildner enthält und in vitro die folgende Freisetzungsgeschwindigkeit aufweist, gemessen unter Anwendung der Ph. Eur. Paddle Method bei 75 U/min in einem Puffer (gemäß Ph. Eur.) bei einem pH-Wert von 6,8 bei 37 °C und unter UV-spektrometrischer Detektion:
3-35 Gew.-% 1-Dimethylamino-3-(3-methoxy-phenyl)-2-methyl-pentan-3-ol (bezogen auf 100 Gew.-% Wirkstoff) nach 0,5 Stunden freigesetzt,
5-50 Gew.-% 1-Dimethylamino-3-(3-methoxy-phenyl)-2-methyl-pentan-3-ol nach 1 Stunde freigesetzt,
10-75 Gew.-% 1-Dimethylamino-3-(3-methoxy-phenyl)-2-methyl-pentan-3-ol nach 2 Stunden freigesetzt,
15-82 Gew.-% 1-Dimethylamino-3-(3-methoxy-phenyl)-2-methyl-pentan-3-ol nach 3 Stunden freigesetzt,
30-97 Gew.-% 1-Dimethylamino-3-(3-methoxy-phenyl)-2-methyl-pentan-3-ol nach 6 Stunden freigesetzt,
mehr als 50 Gew.-% 1-Dimethylamino-3-(3-methoxy-phenyl)-2-methyl-pentan-3-ol nach 12 Stunden freigesetzt,
mehr als 70 Gew.-% 1-Dimethylamino-3-(3-methoxy-phenyl)-2-methyl-pentan-3-ol nach 18 Stunden freigesetzt,
mehr als 80 Gew.-% 1-Dimethylamino-3-(3-methoxy-phenyl)-2-methyl-pentan-3-ol nach 24 Stunden freigesetzt.

Es hat sich überraschend gezeigt, daß die erfindungsgemäße Formulierung den Wirkstoff 1-Dimethylamino-3-(3-methoxy-phenyl)-2-methyl-pentan-3-ol bei oraler Verabreichung verzögert freisetzt und sich somit für eine Verabreichung im Abstand von mindestens 12 Stunden eignet. Die erfindungsgemäße Formulierung erlaubt demnach eine Schmerztherapie bzw. auch eine Behandlung von vermehrtem Harndrang bzw. Harninkontinenz, insbesondere Dranginkontinenz und Stressinkontinenz, in deren Verlauf 1-Dimethylamino-3-(3-methoxy-phenyl)-2-methyl-pentan-3-ol nur noch einmal täglich, z.B. im Abstand von 24 h, oder zweimal täglich, vorzugsweise im Abstand von 12 Stunden, verabreicht werden muß, um eine ausreichende Plasmakonzentration des Wirkstoffs zu gewährleisten. Eine entsprechende Wirkdauer und die Aufrechterhaltung ausreichender Blutplasma-Spiegel wird durch Simulationsstudien und experimentelle Untersuchungen belegt.

Besonders überraschend ist dabei, daß die erfindungsgemäße Formulierung nicht nur aufgrund der verzögerten Freisetzung eine langandauerde therapeutische Wirksamkeit über einen relativ langen Zeitraum (mindestens 12 Stunden) gewährleistet, sondern zugleich bei der ersten Einnahme des Arzneimittels ein rasches Anfluten des Wirkstoffs im Plasma ermöglicht, was zu einer raschen Schmerzlinderung beim Patienten führt ("rapid onset"). Damit kann bei Verabreichung der erfindungsgemäßen Formulierung an einen Schmerzpatienten dessen Schmerz rasch gelindert werden, ohne daß die analgetische Wirkung ebenso rasch wieder nachlassen würde. Die erfindungsgemäße Formulierung vereinigt damit Eigenschaften einer Formulierung mit sofortiger Wirkstofffreisetzung - schnelle Linderung des Schmerzes durch ausreichend hohe Wirkstoffkonzentration kurz nach Gabe des Arzneimittels - mit Eigenschaften einer Formulierung mit verzögerter Freisetzung - langandauernde analgetische Wirkung aufgrund eines ausreichend hohen Wirkstoffspiegels über längere Zeit. Der Schmerzpatient kann somit durch Einnahme des Analgetikums in der erfindungsgemäßen Formulierung seine Schmerzen wirksam akut bekämpfen und zugleich ohne weitere Maßnahmen und lediglich durch regelmäßige Einnahme im Abstand von 12 (oder 24) Stunden effektiv über einen längeren Zeitraum therapieren.

Ebenso günstig ist es auch, dass es durch den durch die Formulierungen erreichten konstanten und ausreichend hohen Wirkstoffspiegel des 1-Dimethylamino-3-(3-methoxy-phenyl)-2-methyl-pentan-3-ol über längere Zeit und zugleich ohne weitere Maßnahmen und lediglich durch regelmäßige Einnahme im Abstand von 12 (oder 24) Stunden gelingt, effektiv über einen längeren Zeitraum vermehrten Harndrang bzw. Harninkontinenz, insbesondere Dranginkontinenz und Stressinkontinenz, zu therapieren, zu behandeln oder zu lindern.

Der Wirkstoff der erfindungsgemäßen Formulierung ist in einer Matrix mit verzögerter Freisetzung enthalten. Es ist jedoch auch denkbar, daß der Wirkstoff in einer Matrix mit üblichem Freisetzungsverhalten enthalten ist und die verzögerte Freisetzung durch eine Überzugsretardierung erreicht wird.

Für den Fall, daß die erfindungsgemäße Formulierung eine Matrix mit verzögerter Freisetzung enthält, weist die Matrix 1-80 Gew.-% eines oder mehrerer hydrophiler oder hydrophober Polymere als pharmazeutisch annehmbare Matrixbildner auf, beispielsweise Gummis, Celluloseether, Celluloseester, Acrylharze, von Proteinen abgeleitete Materialien, Fette, Wachse, Fettalkohole oder Fettsäureester. Bei der Verwendung hydrophiler Polymere als Matrixbildner ist es bevorzugt, daß die Matrix 5 bis 80 Gew.-% Matrixbildner aufweist.

Ein weiterer Gegenstand der vorliegenden Erfindung ist eine pharmazeutische Formulierung, die 1-Dimethylamino-3-(3-methoxyphenyl)-2-methyl-pentan-3-ol oder ein pharmazeutisch annehmbares Salz davon in einer Matrix mit verzögerter Wirkstofffreisetzung enthält, wobei die Matrix 1 bis 80 Gew.-%, insbesondere 5 bis 80 Gew.-%, eines oder mehrerer hydrophiler oder hydrophober Polymeren als pharmazeutisch annehmbaren Matrixbildner enthält und die dadurch gekennzeichnet ist, daß sie als pharmazeutisch annehmbaren Matrixbildner Celluloseether und/oder Celluloseester aufweist, der/die in einer 2 gew.-% wäßrigen Lösung bei 20 °C eine Viskosität von 3.000 bis 150.000 mPa·s aufweist/aufweisen. (Die Viskositätsbestimmung erfolgt dabei mittels Kapillar-Viskosimetrie nach Pharm. Eu.). Die Zusammensetzungen weisen das oben angegebene erfindungsgemäße Freisetzungsprofil auf.

Vorzugsweise werden als pharmazeutisch akzeptable Matrixbildner Celluloseether und/oder Celluloseester eingesetzt, die in einer 2 gew.-%igen wäßrigen Lösung bei 20° C eine Viskosität zwischen 10.000, insbesondere 50.000 mPa·s, und 150.000 mPa·s besitzen.

Besonders geeignete pharmazeutisch akzeptable Matrixbildner sind ausgewählt aus der Gruppe der Hydroxypropylmethylcellulosen (HPMC), Hydroxyethylcellulosen, Hydroxypropylcellulosen (HPC), Methylcellulosen, Ethylcellulosen und Carboxymethylcellulosen und sind insbesondere ausgewählt aus der Gruppe der HPMCs, Hydroxyethylcellulosen und HPCs. Am meisten bevorzugt sind HPMCs mit einer Viskosität von ca. 100.000 mPa·s, gemessen in einer 2 gew.-%igen wäßrigen Lösung bei 20° C.

Der Wirkstoff 1-Dimethylamino-3-(3-methoxy-phenyl)-2-methyl-pentan-3-ol kann als solches, d.h. als freie Base, aber auch in Form eines pharmazeutisch akzeptablen Salzes, beispielsweise als Hydrochlorid, vorliegen. Die Herstellung der freien Base ist aus der EP 0 693 475 A1 bekannt. Soweit in der EP 0 693 475 A1 nicht auch die Herstellung pharmazeutisch annehmbarer Salze - wie des Hydrochlorids - offenbart ist, sind diese mittels im Stand der Technik allgemein bekannter Verfahren ausgehend von der freien Base erhältlich.

1-Dimethylamino-3-(3-methoxy-phenyl)-2-methyl-pentan-3-ol weist zwei Asymmetriezentren auf, so daß die Verbindung in Form von vier verschiedenen Stereoisomeren vorliegen kann. In der erfindungsgemäßen Formulierung kann 1-Dimethylamino-3-(3-methoxy-phenyl)-2-methyl-pentan-3-ol als Gemisch aller vier Diastereomeren in beliebigem Mischungsverhältnis, aber auch als Gemisch von zwei oder drei der vier Stereoisomeren oder in stereoisomerenreiner Form vorliegen. Bevorzugte Stereoisomeren sind hierbei (+)-(2R,3R)-1-Dimethylamino-3-(3-methoxyphenyl)-2-methyl-pentan-3-ol und (-)-(1S,2S)-1-Dimethylamino-3-(3-methoxy-phenyl)-2-methyl-pentan-3-ol, die in der erfindungsgemäßen Formulierung als Gemisch, insbesondere als 1:1-Gemisch (Racemat), oder besonders bevorzugt in isomerenreiner Form vorliegen können. Unter "Wirkstoff" ist daher für die Zwecke der vorliegenden Erfindung 1-Dimethylamino-3-(3-methoxy-phenyl)-2-methyl-pentan-3-ol als Mischung verschiedener seiner Stereoisomeren oder als eines seiner reinen Stereoisomeren, jeweils als freie Base oder in Form eines pharmazeutisch annehmbaren Salzes, zu verstehen.

In den erfindungsgemäßen Arzneimitteln liegt der verzögert freizusetzende Wirkstoffgehalt vorzugsweise zwischen 0,5 und 85 Gew.-% und der Gehalt an pharmazeutisch akzeptablem Matrixbildner zwischen 8 und 40 Gew.-%. Besonders bevorzugt sind Arzneimittel mit einem verzögert freizusetzenden Wirkstoffgehalt zwischen 3 und 70 Gew.-%, insbesondere zwischen 8 und 66 Gew.-%, und einem Gehalt an pharmazeutisch akzeptablem Matrixbildner zwischen 10 und 35 Gew.-%, insbesondere zwischen 10 und 30 Gew.-%. Wird als Wirkstoff das enantiomerenreine (+)-(2R,3R)-1-Dimethylamino-3-(3-methoxy-phenyl)-2-methyl-pentan-3-ol (oder ein Gemisch der (+)- und (-)-Enantiomeren mit großem Überschuß des (+)-Enantiomeren) verwendet, ist es besonders bevorzugt, daß der Wirkstoffgehalt an der unteren Grenze liegt, d.h. zwischen 0,5 und 25 Gew.-% (bezogen auf das Gesamtgewicht). Wird als Wirkstoff das enantiomerenreine (-)-(2S,3S)-1-Dimethylamino-3-(3-methoxy-phenyl)-2-methyl-pentan-3-ol (oder ein Gemisch der (+)- und (-)-Enantiomeren mit großem Überschuß des (-)-Enantiomeren) verwendet, ist es besonders bevorzugt, daß der Wirkstoffgehalt zwischen 16 und 66 Gew.-% liegt.

Weitere Bestandteile der Matrix der erfindungsgemäßen Formulierung können ggf. verdauliche langkettige (d.h. mit 8 bis 50 C-Atomen, bevorzugt 12 bis 40 C-Atomen) unsubstiuierte oder substituierte Kohlenwasserstoffe, wie z.B. Fettalkohole, Fettsäurenglycerylester, Mineral- und Pflanzenöle sowie Wachse sein, wobei Kohlenwasserstoffe mit einem Schmelzpunkt zwischen 25° und 90° C bevorzugt sind. Insbesondere sind Fettalkohole bevorzugt, ganz besonders Laurylalkohol, Myristylalkohol, Stearylalkohol, Cetylalkohol und Cetylstearylalkohol. Ihr Gehalt in der Matrix beträgt 0 bis 60 Gew.-%. Alternativ oder zusätzlich können auch Polyethylenglycole mit einem Gehalt von 0 bis 60 Gew.-% in der Matrix enthalten sein.

In den erfindungsgemäßen pharmazeutischen Formulierungen können ferner als weitere Bestandteile pharmazeutisch gebräuchliche Hilfsstoffe wie Füllstoffe, beispielsweise Lactose, mikrokristalline Cellulose (MCC) oder Calciumhydrogenphosphat, sowie Gleit-, Schmier- und Fließregulierungsmittel, beispielsweise Talkum, Magnesiumstearat, Stearinsäure und/oder hochdisperses Siliciumdioxid, enthalten sein, deren Gesamtgehalt in der Tablette zwischen 0 und 80 Gew.-%, vorzugsweise zwischen 5 und 65 Gew.-% liegt.

Vielfach ist die Freisetzungsgeschwindigkeit eines Wirkstoffes aus einer Darreichungsform vom pH-Wert des Freisetzungsmediums abhängig. Dieser kann während der Gastrointestinalpassage des Arzneimittels in einem pH-Wert-Bereich von unter 1 bis etwa 8 schwanken. Diese Schwankungen können von einer einnehmenden Person zur anderen verschieden sein. Auch kann bei ein und derselben Person von einer Einnahme zur anderen ein unterschiedliches pH-Wert-Zeit-Profil während der Gastrointestinalpassage gegeben sein. Ist die Freisetzungsgeschwindigkeit des Wirkstoffes aus dem Arzneimittel vom pH-Wert abhängig, so kann dies zu unterschiedlichen Freisetzungsgeschwindigkeiten in vivo und damit unterschiedlicher Bioverfügbarkeit führen. Die Freisetzungsprofile des Wirkstoffs (in Form der Base oder eines seiner pharmazeutisch akzeptablen Salze) aus einer erfindungsgemäßen pharmazeutischen Formulierung sind jedoch überraschenderweise unabhängig vom pH-Wert, wie er physiologisch während der Gastrointestinalpassage auftreten kann. Die Freisetzungsprofile bei einem Umgebungs-pH-Wert von 1,2, 4,0 und 6,8 sind sowohl untereinander identisch als auch im Vergleich zur Freisetzung während eines pH-Wert-Zeit-Profils von pH 1,2 über pH 2,3 und pH 6,8 bis zu pH 7,2.

Es hat sich gezeigt, daß es für die Erreichung der verzögerten Wirkstofffreisetzung aus der bevorzugt in Tablettenform vorliegenden erfindungsgemäßen Formulierung unerheblich ist, ob bei ansonsten unveränderten Abmessungen und unveränderter Zusammensetzung der Tablette, bezogen auf den Wirkstoff, den Matrixbildner und die fakultativen Bestandteile, als Füllstoff ein wasserlöslicher Füllstoff, beispielsweise Lactose, ein unlöslicher, in wäßrigem Medium nicht quellender Füllstoff, beispielsweise Calciumhydrogenphosphat, oder ein unlöslicher, in wäßrigem Medium quellender Füllstoff, beispielsweise mikrokristalline Cellulose, eingesetzt wird. Alle derartigen Arzneimittel zeigen ein einander entsprechendes Freisetzungsverhalten.

Überraschend ist ferner, daß in den erfindungsgemäßen Zusammensetzungen bei gegebener Wirkstoffmenge die Menge an Matrixbildner und die Menge der fakultativen Bestandteile jeweils über einen relativ großen Bereich variieren können, ohne daß die therapeutische Wirksamkeit von mindestens 12 h bzw. bei zweimal täglicher Applikation in Frage gestellt werden würde (solange die oben angegebenen Mengengrenzen für Wirkstoff, Matrixbildner und die weiteren, fakultativen Bestandteile eingehalten werden). Eine Wirksamkeit über mindestens 12 h ist z.B. bei einem Wirkstoff-Gehalt von ca. 32,25 Gew.-% (bezogen auf das Gewicht der Gesamtzusammensetzung) sowohl in einer Zusammensetzung aus ca. 12,9 Gew.-% HPMC mit einer Viskosität von 100.000 mPa·s als Matrixbildner und einem Gehalt an beispielsweise MCC als Füllstoff von ca. 52,6 Gew.-% als auch in einer Zusammensetzung aus ca. 25,8 Gew.-% derselben HPMC und ca. 39,7 Gew.-% MCC (oder Lactose-Monohydrat) bei sonst gleichen Mengen an Gleit-, Schmier- und Fließregulationsmitteln gewährleistet. Vergleichbares gilt für erfindungsgemäße Zusammensetzungen mit einem höheren oder geringeren Wirkstoff-Gehalt innerhalb der angegebenen Grenzen.

Außerordentlich überraschend ist auch die Beobachtung, daß bei Verabreichung der erfindungsgemäßen pharmazeutischen Formulierungen mit verzögerter Wirkstofffreisetzung bei menschlichen Versuchspersonen trotz des hohen First-pass-Effekts für den Wirkstoff wider Erwarten eine im Vergleich zu Formulierungen mit sofortiger Wirkstofffreisetzung unveränderte Bioverfügbarkeit erreicht wird.

Es sind ferner solche erfindungsgemäßen Zusammensetzungen bevorzugt, deren tₘₐₓ-Wert im Plasmakonzentrations-Zeit-Diagramm in vivo zwischen 2 und 10 h, insbesondere zwischen 3,5 und 6 h und ganz besonders bevorzugt zwischen 4 und 5,5 h nach oraler Verabreichung der Zusammensetzung beträgt, d.h. deren Peak-Plasma-Level in den genannten Zeiträumen auftritt.

Die erfindungsgemäße Formulierung enthält den Wirkstoff 1-Dimethylamino-3-(3-methoxy-phenyl)-2-methyl-pentan-3-ol als solches und/oder als pharmazeutisch annehmbares Salz in einer Menge von üblicherweise 2,5 bis 800 mg, insbesondere 5 bis 400 mg, ganz besonders bevorzugt 10 bis 250 mg (Gewicht des Wirkstoffs 1-Dimethylamino-3-(3-methoxy-phenyl)-2-methyl-pentan-3-ol als Hydrochlorid) pro Dosierungseinheit, wobei das Freisetzungsverhalten der erfindungsgemäßen Formulierung durch die exakte Menge des Wirkstoffs nicht beeinflußt wird, solange die oben angegebenen Gehaltsgrenzen eingehalten werden. Es ist bevorzugt, wenn das wirkstärkere (+)-(2R,3R)-1-Dimethylamino-3-(3-methoxy-phenyl)-2-methyl-pentan-3-ol in einer Menge von 2,5 bis 80 mg, insbesondere 5 bis 40 mg und ganz besonders bevorzugt in einer Menge von 10 bis 25 mg Wirkstoff (bezogen auf das Hydrochlorid) in den erfindungsgemäßen Formulierungen vorliegt, und zwar unter der Maßgabe, daß die oben angegebenen Gehaltsgrenzen eingehalten werden.

Pharmazeutisch annehmbare (oder akzeptable) Salze des Wirkstoffs sind im Sinne dieser Erfindung solche Salze des Wirkstoffs, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Die erfindungsgemäßen pharmazeutischen Formulierungen können sowohl als einfache Tablette als auch als überzogene Tablette, beispielsweise als Filmtablette oder Dragee vorliegen. Üblicherweise sind die Tabletten rund und bikonvex; möglich sind auch oblonge Tablettenformen, die eine Teilbarkeit der Tablette gestatten. Ferner sind auch Granulate, Spheroide, Pellets oder Mikrokapseln möglich, die in Sachets oder Kapseln gefüllt werden oder zu zerfallenden Tabletten verpreßt werden können.

Für die überzogenen Tabletten können ein oder mehrere Überzugsschichten verwendet werden. Als Überzugsmaterial eignen sich bekannte Hydroxypropylmethylcellulosen mit niedriger Viskosität von ca. 1 bis 100 mPa·s und niedrigem Molekulargewicht von < 10.000 (z.B. Pharmacoat 606 mit einer Viskosität von 6 mPa·s in einer 2 gew.-%igen wäßr. Lösung bei 20 °C), die das Freisetzungsprofil der erfindungsgemäßen Arzneimittel nur geringfügig beeinflussen. Dem Fachmann bekannte Diffusionsüberzüge, beispielsweise auf Basis von quellbaren, aber wasserunlöslichen Poly(meth)acrylaten, führen zu einer Modulation der Verzögerung der Wirkstofffreisetzungen aus erfindungsgemäßen pharmazeutischen Formulierungen. Der wirkstoffhaltige, den Wirkstoff retardiert freisetzende Tablettenkern mit einem Wirkstoffgehalt vorzugsweise zwischen 0,5 und 85 Gew.-%, besonders bevorzugt zwischen 3 und 70 Gew.-% und ganz besonders bevorzugt zwischen 8 und 66 Gew.-%, kann mit zusätzlichem Wirkstoff, der nicht retardiert als Initialdosis freigesetzt wird, durch verschiedene, dem Fachmann bekannte Verfahren, beispielsweise Dragieren, Aufsprühen aus Lösungen oder Suspensionen oder durch Pulverauftragverfahren, umhüllt sein, ohne daß das für die gewünschte verzögerte Freisetzung bei gleichzeitigem raschen Anfluten des Wirkstoffs zur schnellen Schmerzlinderung bei erster Gabe der erfindungsgemäßen pharmazeutischen Formulierung zwingend erforderlich ist. Weitere Ausführungsformen stellen Mehrschicht- und Manteltabletten dar, bei denen 1-Dimethylamino-3-(3-methoxy-phenyl)-2-methyl-pentan-3-ol oder eines seiner pharmzeutisch akzeptablen Salze in einer oder mehreren Schichten der Mehrschichttablette mit einem Wirkstoffgehalt vorzugsweise zwischen 0,5 und 85 Gew.-%, besonders bevorzugt zwischen 3 und 70 Gew.-% und ganz besonders bevorzugt zwischen 8 und 66 Gew.-% bzw. im Kern der Manteltablette mit einem Wirkstoffgehalt vorzugsweise zwischen 0,5 und 85 Gew.-%, besonders bevorzugt zwischen 3 und 70 Gew.-% und ganz besonders bevorzugt zwischen 8 und 66 Gew.-% durch einen pharmazeutisch akzeptablen Matrixbildner retardiert freigesetzt wird und die Freisetzung des Wirkstoffs in einer oder mehreren Schichten der Mehrschichttablette bzw. der äußeren Mantelschicht der Manteltabletten unretardiert erfolgt. Mehrschicht- und Manteltabletten können ein oder mehrere wirkstofffreie Überzüge enthalten.

An Stelle einer verzögert freisetzenden Matrix in der pharmazeutischen Formulierung mit verzögerter Freisetzung ist auch die Verwendung einer normal freisetzenden Matrix mit einem die Freisetzung des Wirkstoffs retardierenden Überzugs möglich. Dabei kann z.B. der Wirkstoff in einer üblichen Matrix aus mikrokristalliner Cellulose und ggf. weiteren pharmazeutischen Hilfsstoffen, wie etwa Bindemittel, Füllstoffe, Gleit-, Schmier- und Fließregulierungsmittel, enthalten sein, die mit einem Material überzogen bzw. beschichtet werden, welche die verzögerte Freisetzung des Wirkstoffs in wäßrigem Medium steuern. Geeignete Beschichtungsmittel sind z.B. wasserunlösliche Wachse und Polymere, wie Polymethacrylate (Eudragit o.ä.) oder wasserunlösliche Cellulosen, insbesondere Ethylcellulose. Ggf. können im Überzugsmaterial auch wasserlösliche Polymere, wie Polyvinylpyrrolidon, wasserlösliche Cellulosen, wie Hydroxypropylmethylcellulose oder Hydroxypropylcellulose, andere wasserlösliche Mittel, wie Polysorbat 80, oder hydrophile Porenbildner, wie Polyethylenglycol, Lactose oder Mannitol, enthalten sein.

Die erfindungsgemäßen Zusammensetzungen können beispielsweise nach folgendem allgemeinen Verfahren hergestellt werden:
Die Bestandteile der Zusammensetzung (Wirkstoff, Matrixbildner und fakultative Bestandteile) werden der Reihe nach eingewogen und anschließend auf einer üblichen Siebmaschine gesiebt. Hier kann beispielsweise die Siebmaschine Quadro Comil U10 eingesetzt werden, wobei eine gebräuchliche Siebgröße ca. 0,813 mm beträgt. Die Siebung wird anschließend in einem Containermischer gemischt, z.B. in einem Bohle Containermischer; typische Arbeitsbedingungen sind: Dauer ca. 15 min ± 45 s bei einer Drehzahl von 20 ± 1 U/min. Danach wird die erhaltene Pulvermischung auf einer Tablettenpresse zu einer Tablette verpreßt. Hierfür kann z.B eine Tablettenpresse Korsch EKO mit einem drageegewölbten Rundstempel mit 10 mm Durchmesser Verwendung finden. Alternativ kann auch eine Kompaktierung der Pulvermischung und anschließende Siebung (Comill 3 mm Reibschnitzelsieb und anschließend 1,2 mm Rundlochsieb) der Preßlinge erfolgen, wobei das so entstehende Granulat anschließend wie oben beschrieben unter Zusatz von Schmiermittel (z.B. Magnesiumstearat) auf z.B. einer EK0 Tablettenpresse mit 10 mm Rundstempeln verpreßt wird. Die Granulation kann auch durch Naßgranulation auf Basis wäßriger oder organischer Lösungsmittel erfolgen; bevorzugt sind dabei wäßrige Lösungsmittel mit oder ohne geeignete Bindemittel. Das Herstellungsverfahren kann ohne weiteres an die jeweiligen Erfordernisse und die gewünschte Darreichungform nach im Stand der Technik wohlbekannten Vorgehensweisen angepaßt werden.

Die Herstellung erfindungsgemäßer pharmazeutischer Formulierungen ist durch eine hohe Reproduzierbarkeit der Freisetzungseigenschaften der erhaltenen Zusammensetzungen, die 1-Dimethylamino-3-(3-methoxyphenyl)-2-methyl-pentan-3-ol oder eines seiner pharmzeutisch akzeptablen Salze enthalten, gekennzeichnet. Während einer Lagerzeit von mindestens einem Jahr unter den üblichen Lagerungsbedingungen gemäß ICH Q1AR-Stability-Testing-Guideline erweist sich das Freisetzungsprofil erfindungsgemäßer Arzneimittel als stabil.

Bei täglich ein- oder zweimaliger Einnahme einer erfindungsgemäßen pharmazeutischen Formulierung durch den Patienten wird eine gute therapeutische Wirksamkeit bei anhaltend starken Schmerzen aber auch eine gute therapeutische Wirksamkeit in der Behandlung von vermehrtem Harndrang bzw. Harninkontinenz, insbesondere Stressinkontinenz und Dranginkontinenz, sicher erzielt.

Daher ist ein weiterer Gegenstand der Erfindung die Verwendung einer erfindungsgemäßen pharmazeutischen Formulierung oder einer erfindungsgemäßen Tablette zur Herstellung eines Arzneimittels zur Behandlung von Schmerz, insbesondere chronischem, viszeralem, neuropathischem oder akutem Schmerz oder Entzündungs-Schmerz.

Ein weiterer Gegenstand ist die Verwendung von 1-Dimethylamino-3-(3-methoxy-phenyl)-2-methyl-pentan-3-ol zur Herstellung eines Arzneimittels zur Behandlung von Schmerz, insbesondere chronischem, viszeralem, neuropathischem oder akutem Schmerz oder Entzündungs-Schmerz, wobei das 1-Dimethylamino-3-(3-methoxy-phenyl)-2-methyl-pentan-3-ol in einer erfindungsgemäßen pharmazeutischen Formulierung enthalten ist.

Eine weitere Aufgabe der vorliegenden Erfindung war es, Stoffe bzw. pharmazeutische Formulierungen oder Arzneimittel aufzufinden, die zur Behandlung von vermehrtem Harndrang bzw. Harninkontinenz hilfreich sind und die insbesondere wirksame Dosen freisetzen, die geringere Nebenwirkungen und/oder analgetische Wirkungen zeigen als aus dem Stand der Technik bekannt.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskel der Harnröhre. Es treten Mischformen dieser Inkontinenzarten sowie die sogenannte Überflußinkontinenz (z. B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A. J., 1998, Urology 51 (Suppl. 21): 43 - 47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Auch bestimmte Opioide, Diarylmethylpiperazine und -piperidine, sind für diese Indikation in der WO 93/15062 beschrieben.

Bei den hier in Frage kommenden Indikationen ist zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Entsprechend war es daher eine weitere Aufgabe der vorliegenden Erfindung Stoffe bzw. pharmazeutische Formulierungen oder Arzneimittel aufzufinden, die zur Behandlung von vermehrtem Harndrang bzw. Harninkontinenz hilfreich sind.

Überraschenderweise wurde nun gefunden, daß 1-Dimethylamino-3-(3-methoxy-phenyl)-2-methyl-pentan-3-ol und insbesondere die erfindungsgemäßen pharmazeutischen Formulierungen enthaltend 1-Dimethylamino-3-(3-methoxy-phenyl)-2-methyl-pentan-3-ol eine hervorragende Wirkung auf die Blasenfunktion besitzen und demzufolge gut zur Behandlung entsprechender Erkrankungen geeignet sind.

Daher ist ein weiterer Gegenstand der Erfindung die Verwendung einer erfindungsgemäßen pharmazeutischen Formulierung oder einer erfindungsgemäßen Tablette zur Herstellung eines Arzneimittels zur Behandlung von vermehrtem Harndrang bzw. Harninkontinenz.

Ein weiterer Gegenstand der Erfindung ist die Verwendung von 1-Dimethylamino-3-(3-methoxy-phenyl)-2-methyl-pentan-3-ol zur Herstellung eines Arzneimittels zur Behandlung von vermehrtem Harndrang bzw. Harninkontinenz, wobei das 1-Dimethylamino-3-(3-methoxy-phenyl)-2-methyl-pentan-3-ol in einer erfindungsgemäßen pharmazeutischen Formulierung enthalten ist.

### Beispiele

Die Beispiele dienen der Illustration der vorliegenden Erfindung und bevorzugter Ausführungsformen, sollen aber ihren Schutzumfang nicht beschränken.

### Beispiel 1

Matrixtabletten mit folgender Zusammensetzung pro Tablette

| | |
|---|---|
| 1-Dimethylamino-3-(3-methoxy-phenyl)-2-methyl-pentan-3-ol; Hydrochlorid | 5mg |
| Hydroxypropylmethylcellulose (Metolose 90 SH 100.000 von Fa. Fa. Shinetsu), 100.000 mPa·s | 80 mg |
| Mikrokristalline Cellulose (Avicel PH 101 von Fa. FA. FMC) | 50 mg |
| Lactose Monhydrat (Lactose 200 von Fa. Meggle) | 169 mg |
| Hochdisperses Siliciumdioxid | 3 mg |
| Magnesiumstearat | 3 mg |
| Gesamtmenge | 310 mg |

wurden in einer Ansatzgröße von 2000 Tabletten in folgender Weise hergestellt:
Alle Bestandteile wurden eingewogen und auf einer Siebmaschine Quadro Comil U10 unter Verwendung einer Siebgröße von 0,813 mm gesiebt, in einem Continermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Die Freisetzung in vitro wurde bestimmt unter Anwendung der Ph. Eur. Paddle Method bei 75 U/min in 900 ml Puffer pH 6,8 nach Ph. Eur. bei 37 °C und mit UV-spektrometrischem Nachweis und ist in folgender Tabelle wiedergegeben.

| **Zeit [min]** | **Freigesetzte Gesamtmenge des Wirkstoffs [%]** |
|---|---|
| 0 | 0 |
| 30 | 17 |
| 240 | 75 |
| 480 | 95 |
| 720 | 100 |

### Beispiel 2

Matrixtabletten mit folgender Zusammensetzung pro Tablette

| | |
|---|---|
| 1-Dimethylamino-3-(3-methoxy-phenyl)-2-methyl-pentan-3-ol; Hydrochlorid | 50mg |
| Hydroxypropylmethylcellulose (Metolose 90 SH 100.000 von Fa. Fa. Shinetsu), 100.000 mPa·s | 80 mg |
| Mikrokristalline Cellulose (Avicel PH 101 von Fa. FA. FMC) | 174 mg |
| Hochdisperses Siliciumdioxid | 3 mg |
| Magnesiumstearat | 3 mg |
| Gesamtmenge | 310 mg |

wurden in einer Ansatzgröße von 2000 Tabletten in folgender Weise hergestellt:
Alle Bestandteile wurden eingewogen und auf einer Siebmaschine Quadro Comil U10 unter Verwendung einer Siebgröße von 0,813 mm gesiebt, in einem Continermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Die Freisetzung in vitro wurde bestimmt unter Anwendung der Ph. Eur. Paddle Method bei 75 U/min in 900 ml Puffer pH 6,8 nach Ph. Eur. bei 37 °C und mit UV-spektrometrischem Nachweis und ist in folgender Tabelle wiedergegeben.

| **Zeit [min]** | **Freigesetzte Gesamtmenge des Wirkstoffs [%]** |
|---|---|
| 0 | 0 |
| 30 | 20 |
| 240 | 63 |
| 480 | 81 |
| 720 | 91 |

### Beispiel 3

Matrixtabletten mit folgender Zusammensetzung pro Tablette

| | |
|---|---|
| 1-Dimethylamino-3-(3-methoxy-phenyl)-2-methyl-pentan-3-ol; Hydrochlorid | 100mg |
| Hydroxypropylmethylcellulose (Metolose 90 SH 100.000 von Fa. Fa. Shinetsu), 100.000 mPa·s | 80 mg |
| Mikrokristalline Cellulose (Avicel PH 101 von Fa. FA. FMC) | 94 mg |
| Lactose Monhydrat (Lactose 200 von Fa. Meggle) | 30 mg |
| Hochdisperses Siliciumdioxid | 3 mg |
| Magnesiumstearat | 3 mg |
| Gesamtmenge | 310 mg |

wurden in einer Ansatzgröße von 2000 Tabletten in folgender Weise hergestellt:
Alle Bestandteile wurden eingewogen und auf einer Siebmaschine Quadro Comil U10 unter Verwendung einer Siebgröße von 0,813 mm gesiebt, in einem Continermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Die Freisetzung in vitro wurde bestimmt unter Anwendung der Ph. Eur. Paddle Method bei 75 U/min in 900 ml Puffer pH 6,8 nach Ph. Eur. bei 37 °C und mit UV-spektrometrischem Nachweis und ist in folgender Tabelle wiedergegeben.

| **Zeit [min]** | **Freigesetzte Gesamtmenge des Wirkstoffs [%]** |
|---|---|
| 0 | 0 |
| 30 | 22 |
| 240 | 69 |
| 480 | 88 |
| 720 | 96 |

Tabletten gemäß Beispiel 3 wurden 3 Monate bei 40°C und 75% relativer Luftfeuchtigkeit, 9 Monate bei 25°C und 9 Monate bei 30°C (Lagerbedingungen gemäß ICH) gelagert. Anschließend wurde erneut die Freisetzung in vitro unter Anwendung der Ph. Eur. Paddle Method bei 75 U/min in 900 ml Puffer pH 6,8 nach Ph. Eur. bei 37 °C und mit UV-spektrometrischem Nachweis überprüft und ist in folgender Tabelle wiedergegeben:

| **Zeit [min]** | **Lagerbedingungen** | | |
|---|---|---|---|
| | **9 Monate/25°C** | **9 Monate/30°C** | **3 Monate/40°C/ 75% rel. Feuchte** |
| | **Freigesetzte Gesamtmenge des Wirkstoffs [%]** | | |
| 0 | 0 | 0 | 0 |
| 30 | 21 | 21 | 21 |
| 240 | 73 | 72 | 77 |
| 480 | 93 | 92 | 94 |
| 720 | 100 | 99 | 100 |

### Beispiel 4

Matrixtabletten mit folgender Zusammensetzung pro Tablette

| | |
|---|---|
| (-)-(1R,2R)-1-Dimethylamino-3-(3-methoxy-phenyl)-2-methyl-pentan-3-ol-Hydrochlorid | 100 mg |
| Cellactose (Fa. Meggle) | 72,5 mg |
| Hydroxyethylcellulose (Natrosol 250 HX von Fa. Herkules) | 12,5 mg |
| Cutina HR (Fa. Henkel) | 150 mg |
| Talkum | 3 mg |
| Magnesiumstearat | 2 mg |
| Gesamtmenge | 340 mg |

wurden in einer Ansatzgröße von 200 Tabletten wie folgt hergestellt:
Der Wirkstoff, Cellactose, Natrosol und Cutina wurden gemischt, dann im Trockenschrank auf 80 °C erhitzt und in einem Kenwood Chef-Küchenmischer granuliert. Das abgekühlte Granulat wurde durch ein 1 mm-Sieb gesiebt. Nach Abmischen mit Magnesiumstearat und Talkum wurde das Granulat auf einer EK 0 Exzeterpresse (Korsch) zu 6 x 15 mm großen Oblong-Tabletten mit Bruckkerbe verpreßt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 10 |
| 240 | 53 |
| 480 | 69 |
| 720 | 80 |
| 900 | 98 |

### Beispiel 5

Pellets folgender Zusammensetzung

| | |
|---|---|
| (-)-(1R,2R)-1-Dimethylamino-3-(3-methoxy-phenyl)-2-methyl-pentan-3-ol-Hydrochlorid | 100 mg |
| Niedrigsubstituierte Hydroxypropylcellulose(L-HPC LH 31 von Fa. Shinetsu | 75 mg |
| Aquacoat (wäßr. Ethylcellulose-Dispersion; von Fa. FMC) (als Trockensubstanz gerechnet) | 20 mg |
| Mikrokristalline Cellulose (Avicel PH 101, Fa. FMC) | 75 mg |
| Dibutylsebacat (DBS) | 4 mg |
| Tween 80 | 0,4 mg |
| Gesamtmenge | 274,4 mg |

wurden wie folgt hergestellt:
Der Wirkstoff, Avicel und L-HPC wurden in einem Planetenmischer (Kenwood K-Mischer) 10 min gemischt und anschließend mit Wasser granuliert. Das feuchte Granulat wurde im Nica-Extruder mit einer 0,8 x 0,8 mm Matrize extrudiert und dann 10 min im Nica-Spheronizer bei 500 U/min ausgerundet (Beladung 1 kg). Die Pellets wurden über Nacht im Trockenschrank bei 50 °C getrocknet und anschließend in Siebfraktionen klassiert.
Pellets der Größe 0,6-1,0 mm (Ausbeute ca. 95%) wurden in der WSG (Glatt GPCG1 mit Wurstereinsatz) bei Zulufttemperaturen von 60 °C (Produkttemperatur 40 °C) mit einer wäßrigen Dispersion aus Aquacoat und DBS (20 %, berechnet auf Aquacoat Feststoffgehalt) überzogen, so daß sie eine Gewichtszunahme von 9,8 % (bezogen auf das Ausgangsgewicht) aufwiesen. Die Herstellung der Dispersion erfolgte gemäß Herstellerangaben (FA. FMC), wobei das DBS zusammen mit Tween 80 in einer Teilmenge des Wassers homogenisiert und dann zur verdünnten Aquacoat-Dispersion hinzugegeben wurde. Die fertige Dispersion hatte einen Feststoffgehalt von 20 Gew.-% und wurde mind. 3h gerührt. Die überzogenen Pellets wurden in der WSG getrocknet und im Trockenschrank getempert (2h bei 60 °C). Die Prüfung der Freisetzung erfolgte analog Beispiel 1, jedoch nach der Körbchenmethode bei 100 U/min.

| **Zeit [min]** | **Freigesetzte Gesamtmenge des Wirkstoffs [%]** |
|---|---|
| 0 | 0 |
| 30 | 2 |
| 240 | 29 |
| 480 | 67 |
| 720 | 78 |
| 900 | 89 |
| 1080 | 101 |

### Beispiel 6: Liste der getesteten Substanzen:

Es folgt eine Liste der auf ihre Wirksamkeit getesteten Verbindungen:

| **Name** | **Verbdg. Nr.** |
|---|---|
| (2RS,3RS)-1-Dimethylamino-3-(3-methoxy-phenyl)-2-methyl-pentan-3-ol, Hydrochlorid | **1** |
| (+)-(2R,3R)-1-Dimethylamino-3-(3-methoxy-phenyl)-2-methyl-pentan-3-ol, Hydrochlorid | **2** |
| (-)-(2S,3S)-1-Dimethylamino-3-(3-methoxy-phenyl)-2-methyl-pentan-3-ol, Hydrochlorid | **21** |

### Beispiel 8: Testsystem Cystometrie an der wachen naiven Ratte

Es wurden cystometrische Untersuchungen an naiven, weiblichen Sprague-Dawley-Ratten nach der Methode von Ishizuka et. al. ((1997), Naunyn-Schmiedeberg's Arch. Pharmacol. 355: 787 - 793) durchgeführt. Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet. Nach einer Stabilisierungsphase wurde eine 20minütige Phase aufgezeichnet, die durch normale, reproduzierbare Miktionszyklen gekennzeichnet war. Es wurden unter anderem die folgenden Parameter bestimmt:
■ Schwellendruck (threshold pressure TP, Blasendruck unmittelbar vor Miktion),
■ Blasenkapazität (bladder capacity BC, Restvolumen nach vorhergehender Miktion plus Volumen der infudierten Lösung während der Füllungsphase),
■ Interkontraktionsintervall (inter-contraction interval (ICI), das Zeitintervall zwischen den Miktionen).

Eine Erhöhung des Schwellendrucks (TP) zeigt eine wichtige therapeutische Wirkung bei einer der erfindungsgemässen Indikationen an. Auch das Interkontraktionsintervall (ICI) ist ein wichtiger Parameter zur Messung der physiologischen Wirksamkeit eines Stoffes in der Behandlung der Harninkontinenz, ebenso wie die Blasenkapazität (BC). Dabei ist es für eine Wirksamkeit aufgrund der sehr heterogenen Ursachen für die Symptomatik dieser Erkrankungsbilder nicht nötig, alle drei Parameter positiv zu beeinflussen. Es genügt daher völlig, wenn nur in einem dieser Parameter eine posive Wirkung festzustellen ist, um in der Harninkontinenz oder vermehrtem Harndrang einsetzbar zu sein.

Nach der Aufzeichnung von drei reproduzierbaren Miktionszyklen als Vorwert, wurden die Testsubstanzen **1** (1,0 mg/kg), **2** (0,1; 0,3 und 0,5 mg/kg) und **21** (0,5 mg/kg) im Vehikel = 0.9 % NaCl i.v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 1).

| **Verbindung:** | **TP** | **BC** | **ICI** |
|---|---|---|---|
| (Konzentration) | threshold pressure | bladder capacity | intercontraction interval |
| **1** | | | |
| 1,0 mg/kg iv | +94 % ** | +31 % *** | +42 % |
| (n=9) | | | |
| **2** | | | |
| 0,1 mg/kg iv | +28,5 % ** | +7,8 % | +15,6 % |
| (n=5) | | | |
| 0,3 mg/kg iv | +122 %** | +33 %* | +28 %* |
| (n=8) | | | |
| 0,5 mg/kg iv | +77,5 %** | +20,6 %* | +28,6 %** |
| (n=9) | | | |
| **21** | | | |
| 0,5 mg/kg iv | -1,1 % | +3 % | +10 % |
| (n=8) | | | |

| | | | |
|---|---|---|---|
| **Tabelle 1:** Beeinflussung der cystometrischen Parameter durch die Testsubstanzen (Veränderung zum Vorwert [%]); n entspricht der Anzahl der Versuchstiere; Signifikanz (Student T-Test): * p < 0.05; ** p < 0.01; *** p < 0.001. | | | |

Die untersuchten Substanzen zeigen eine positive Wirkung auf die Blasenregulation und sind somit geeignet zur Behandlung der Harninkontinenz.

Unter anderem zeigt sich, daß von den Enantiomeren der racemischen Verbindung 1 das (+)- Enantiomere (Verbindung 2) sehr wirksam ist (und damit eine besonders bevorzugte Verbindung dieser Erfindung ist), während das (-)-Enantiomere (Verbindung 21) nicht so stark zur Wirkung beisteuert.

## Patentansprüche

1. Pharmazeutische Formulierung mit verzögerter Freisetzung, die 1-Dimethylamino-3-(3-methoxy-phenyl)-2-methyl-pentan-3-ol oder ein pharmazeutisch annehmbares Salz davon in einer Matrix mit verzögerter Wirkstofffreisetzung enthält,
wobei die Matrix 1 bis 80 Gew.-% eines oder mehrerer hydrophiler oder hydrophober Polymeren als pharmazeutisch annehmbaren Matrixbildner enthält und
in vitro die folgende Freisetzungsgeschwindigkeit aufweist, gemessen unter Anwendung der Ph. Eur. Paddle Method bei 75 U/min in einem Puffer (gemäß Ph. Eur.) bei einem pH-Wert von 6,8 bei 37 °C und unter UV-spektrometrischer Detektion:
3-35 Gew.-% (bezogen auf 100 Gew.-% Wirkstoff) 1-Dimethylamino-3-(3-methoxy-phenyl)-2-methyl-pentan-3-ol nach 0,5 Stunden freigesetzt,
5-50 Gew.-% 1-Dimethylamino-3-(3-methoxy-phenyl)-2-methyl-pentan-3-ol nach 1 Stunde freigesetzt,
10-75 Gew.-% 1-Dimethylamino-3-(3-methoxy-phenyl)-2-methyl-pentan-3-ol nach 2 Stunden freigesetzt,
15-82 Gew.-% 1-Dimethylamino-3-(3-methoxy-phenyl)-2-methyl-pentan-3-ol nach 3 Stunden freigesetzt,
30-97 Gew.-% 1-Dimethylamino-3-(3-methoxy-phenyl)-2-methyl-pentan-3-ol nach 6 Stunden freigesetzt,
mehr als 50 Gew.-% 1-Dimethylamino-3-(3-methoxy-phenyl)-2-methyl-pentan-3-ol nach 12 Stunden freigesetzt,
mehr als 70 Gew.-% 1-Dimethylamino-3-(3-methoxy-phenyl)-2-methyl-pentan-3-ol nach 18 Stunden freigesetzt,
mehr als 80 Gew.-% 1-Dimethylamino-3-(3-methoxy-phenyl)-2-methyl-pentan-3-ol nach 24 Stunden freigesetzt.

2. Pharmazeutische Formulierung mit verzögerter Freisetzung, die 1-Dimethylamino-3-(3-methoxy-phenyl)-2-methyl-pentan-3-ol oder ein pharmazeutisch annehmbares Salz davon in einer Matrix mit verzögerter Wirkstofffreisetzung enthält, wobei die Matrix 1 bis 80 Gew.-% eines oder mehrerer hydrophiler oder hydrophober Polymeren als pharmazeutisch annehmbaren Matrixbildner enthält und als pharmazeutisch annehmbaren Matrixbildner Celluloseether und/oder Celluloseester aufweist, der/die in einer 2 gew.-% wäßrigen Lösung bei 20 °C eine Viskosität von 3.000 bis 150.000 mPa·s aufweist.

3. Pharmazeutische Zusammensetzung nach einem der Ansprüche 1 bis 2, **dadurch gekennzeichnet, daß** sie als pharmazeutisch annehmbaren Matrixbildner Celluloseether und/oder Celluloseester aufweist, der/die in einer 2 gew.-% Lösung bei 20 °C eine Viskosität von 10.000 bis 150.000 mPas aufweist/aufweisen.

4. Pharmazeutische Zusammensetzung nach einem der Ansprüche 1 bis 3, **dadurch gekennzeichnet, daß** sie als pharmazeutisch annehmbaren Matrixbildner Celluloseether und/oder Celluloseester aufweist, der/die in einer 2 gew.-% Lösung bei 20 °C eine Viskosität von 50.000 bis 150.000 mPa·s aufweist/aufweisen.

5. Pharmazeutische Zusammensetzung nach einem der Ansprüche 1 bis 4, **dadurch gekennzeichnet, daß** sie als pharmazeutisch annehmbaren Matrixbildner mindestens eine Substanz enthält, die aus der Gruppe ausgewählt ist, die Hydroxypropylmethylcellulosen (HPMC), Hydroxyethylcellulosen, Hydroxypropylcellulosen (HPC), Methylcellulosen, Ethylcellulosen und Carboxymethylcellulosen umfaßt.

6. Pharmazeutische Zusammensetzung nach einem der Ansprüche 1 bis 5, **dadurch gekennzeichnet, daß** sie als pharmazeutisch annehmbaren Matrixbildner mindestens eine Substanz enthält, die aus der Gruppe ausgewählt ist, die Hydroxypropylmethylcellulosen, Hydroxyethylcellulosen und Hydroxypropylcellulosen umfaßt.

7. Pharmazeutische Zusammensetzung nach einem der Ansprüche 1 bis 6, **dadurch gekennzeichnet, daß** der Gehalt des verzögert freizusetzenden Wirkstoffs zwischen 0,5 und 85 Gew.-% und der Gehalt an pharmazeutisch akzeptablem Matrixbildner zwischen 8 und 40 Gew.-% liegt.

8. Pharmazeutische Zusammensetzung nach einem der Ansprüche 1 bis 7, **dadurch gekennzeichnet, daß** der Gehalt des verzögert freizusetzenden Wirkstoffs zwischen 3 und 70 Gew.-%, insbesondere zwischen 8 und 66 Gew.-%, und der Gehalt an pharmazeutisch akzeptablem Matrixbildner zwischen 10 und 35 Gew.-%, insbesondere zwischen 10 und 30 Gew.-%, liegt.

9. Pharmazeutische Zusammensetzung nach einem der Ansprüche 1 bis 8, **dadurch gekennzeichnet, daß** der Peak-Plasma-Level des Wirkstoffs in vivo nach 2 h bis 10 h, insbesondere nach 3,5 h bis 6 h erreicht wird.

10. Pharmazeutische Formulierung nach einem der Ansprüche 1 bis 9, **dadurch gekennzeichnet, daß** sie (+)-(2R,3R)-1-Dimethylamino-3-(3-methoxy-phenyl)-2-methyl-pentan-3-ol oder ein pharmazeutisch annehmbares Salz davon enthält.

11. Tablette für die 2-mal tägliche orale Verabreichung von 1-Dimethylamino-3-(3-methoxy-phenyl)-2-methyl-pentan-3-ol, enthaltend eine pharmazeutische Formulierung nach einem der Ansprüche 1 bis 10.

12. Verwendung einer pharmazeutischen Formulierung nach einem der Ansprüche 1 bis 10 oder einer Tablette nach Anspruch 11 zur Herstellung eines Arzneimittels zur Behandlung von vermehrtem Harndrang bzw. Harninkontinenz.

13. Verwendung von 1-Dimethylamino-3-(3-methoxy-phenyl)-2-methyl-pentan-3-ol zur Herstellung eines Arzneimittels zur Behandlung von vermehrtem Harndrang bzw. Harninkontinenz, **dadurch gekennzeichnet, daß** das 1-Dimethylamino-3-(3-methoxy-phenyl)-2-methyl-pentan-3-ol in einer pharmazeutischen Formulierung nach einem der Ansprüche 1 bis 10 enthalten ist.

14. Verwendung einer pharmazeutischen Formulierung nach einem der Ansprüche 1 bis 10 oder einer Tablette nach Anspruch 11 zur Herstellung eines Arzneimittels zur Behandlung von Schmerz, insbesondere chronischem, viszeralem, neuropathischem oder akutem Schmerz oder Entzündungs-Schmerz.

15. Verwendung von 1-Dimethylamino-3-(3-methoxy-phenyl)-2-methyl-pentan-3-ol zur Herstellung eines Arzneimittels zur Behandlung von Schmerz, insbesondere chronischem, viszeralem, neuropathischem oder akutem Schmerz oder Entzündungs-Schmerz, **dadurch gekennzeichnet, daß** das 1-Dimethylamino-3-(3-methoxy-phenyl)-2-methyl-pentan-3-ol in einer pharmazeutischen Formulierung nach einem der Ansprüche 1 bis 10 enthalten ist.

## Claims

1. Pharmaceutical formulation with delayed release, that contains 1-dimethylamino-3-(3-methoxyphenyl)-2-methylpentan-3-ol or a pharmaceutically acceptable salt thereof in a matrix with delayed release of active constituent, wherein the matrix contains 1 to 80 wt.% of one or more hydrophilic or hydrophobic polymers as pharmaceutically acceptable matrix-forming agents, and
exhibits the following *in vitro* release rates measured using the:Ph. Eur. paddle method at 75 rpm in a buffer (according to Ph. Eur.) at a pH value of 6.8 at 37°C and with UV spectrometric detection:
3-35 wt.% of 1-dimethylamino-3-(3-methoxyphenyl)-2-methylpentan-3-ol (referred to 100 wt.% of active constituent) released after 0.5 hour,
5-50 wt.% of 1-dimethylamino-3-(3-methoxyphenyl)-2-methylpentan-3-ol released after 1 hour
10-75 wt.% of 1-dimethylamino-3-(3-methoxyphenyl)-2-methylpentan-3-ol released after 2 hours
15-82 wt.% of 1-dimethylamino-3-(3-methoxyphenyl)-2-methylpentan-3-ol released after 3 hours
30-97 wt.% of 1-dimethylamino-3-(3-methoxyphenyl)-2-methylpentan-3-ol released after 6 hours
more than 50 wt.% of 1-dimethylamino-3-(3-methoxyphenyl)-2-methylpentan-3-ol released after 12 hours,
more than 70 wt.% of 1-dimethylamino-3-(3-methoxyphenyl)-2-methylpentan-3-ol released after 18 hours,
more than 80 wt.% of 1-dimethylamino-3-(3-methoxyphenyl)-2-methylpentan-3-ol released after 24 hours.

2. Pharmaceutical formulation with delayed release, that contains 1-dimethylamino-3-(3-methoxyphenyl)-2-methylpentan-3-ol or a pharmaceutically acceptable salt thereof in a matrix with delayed release of active constituent, wherein the matrix contains 1 to 80 wt.% of one or more hydrophilic or hydrophobic polymers as pharmaceutically acceptable matrix-forming agents and comprises as pharmaceutically acceptable matrix-forming agents cellulose ethers and/or cellulose esters that in a 2 wt.% aqueous solution at 20°C have a viscosity of 3000 to 150,000 mPa·s.

3. Pharmaceutical formulation according to one of claims 1 and 2, **characterised in that** it comprises as pharmaceutically acceptable matrix-forming agents cellulose ethers and/or cellulose esters that in a 2 wt.% solution at 20°C have a viscosity of 10,000 to 150,000 mPa·s.

4. Pharmaceutical formulation according to one of claims 1 to 3, **characterised in that** it comprises as pharmaceutically acceptable matrix-forming agents cellulose ethers and/or cellulose esters that in a 2 wt.% solution at 20°C have a viscosity of 50,000 to 150,000 mPa·s.

5. Pharmaceutical formulation according to one of claims 1 to 4, **characterised in that** it contains as pharmaceutically acceptable matrix-forming agents at least one substance that is selected from the group comprising hydroxypropylmethylcelluloses (HPMC), hydroxyethylcelluloses, hydroxypropylcelluloses (HPC), methylcelluloses, ethylcelluloses and carboxymethylcelluloses.

6. Pharmaceutical formulation according to one of claims 1 to 5, **characterised in that** it contains as pharmaceutically acceptable matrix-forming agents at least one substance that is selected from the group comprising hydroxypropylmethylcelluloses, hydroxyethylcelluloses and hydroxypropylcelluloses.

7. Pharmaceutical formulation according to one of claims 1 to 6, **characterised in that** the content of the delayed release active constituent is between 0.5 and 85 wt.% and the content of pharmaceutically acceptable matrix-forming agent is between 8 and 40 wt.%.

8. Pharmaceutical formulation according to one of claims 1 to 7, **characterised in that** the content of the delayed release active constituent is between 3 and 70 wt.%, in particular between 8 and 66 wt.%, and the content of pharmaceutically acceptable matrix-forming agent is between 10 and 35 wt.%, in particular between 10 and 30 wt.%.

9. Pharmaceutical formulation according to one of claims 1 to 8, **characterised in that** the peak plasma level of the active constituent *in vivo* is reached after 2 hours to 10 hours, in particular after 3.5 hours to 6 hours.

10. Pharmaceutical formulation according to one of claims 1 to 9, **characterised in that** it contains (+)-(2R,3R)-1-dimethylamino-3-(3-methoxyphenyl)-2-methylpentan-3-ol or a pharmaceutically acceptable salt thereof.

11. Tablet for twice daily oral administration of 1-dimethylamino-3-(3-methoxyphenyl)-2-methylpentan-3-ol, containing a pharmaceutical formulation according to one of claims 1 to 10.

12. Use of a pharmaceutical formulation according to one of claims 1 to 10 or a tablet according to claim 11 for the production of a medicament for treating increased urinary urgency or urinary incontinence.

13. Use of 1-dimethylamino-3-(3-methoxyphenyl)-2-methylpentan-3-ol for producing a medicament for treating increased urinary urgency or urinary incontinence, **characterised in that** 1-dimethylamino-3-(3-methoxyphenyl)-2-methylpentan-3-ol is contained in a pharmaceutical formulation according to one of claims 1 to 10.

14. Use of a pharmaceutical formulation according to one of claims 1 to 10 or a tablet according to claim 11 for the production of a medicament for treating pain, in particular chronic, visceral, neuropathic or acute pain or inflammation pain.

15. Use of 1-dimethylamino-3-(3-methoxyphenyl)-2-methylpentan-3-ol for the production of a medicament for treating pain, in particular chronic, visceral, neuropathic or acute pain or inflammation pain, **characterised in that** 1-dimethylamino-3-(3-methoxyphenyl)-2-methylpentan-3-ol is contained in a pharmaceutical formulation according to one of claims 1 to 10.

## Revendications

1. Formulation pharmaceutique à libération retardée, qui contient du 1-diméthylamino-3-(3-méthoxyphényl)-2-méthylpentane-3-ol ou un sel pharmaceutiquement acceptable de ce composé dans une matrice à libération retardée de la substance active, la matrice contenant 1 à 80 % en poids d'un ou plusieurs polymères hydrophiles ou hydrophobes comme substance pharmaceutiquement acceptable formant cette matrice et présentant *in vitro* la vitesse de libération suivante, mesurée d'après la Paddle Method de la Pharmacopée Européenne, de 75 U/min dans un tampon (selon la Pharmacopée Européenne) à un pH de 6,8 à 37°C et avec détection par spectrométrie UV :
3 - 35 % en poids de 1-diméthylamino-3-(3-méthoxyphényl)-2-méthylpentane-3-ol (sur la base de 100 % en poids de substance active) libérés après 0,5 heure,
5 - 50 % en poids de 1-diméthylamino-3-(3-méthoxyphényl)-2-méthylpentane-3-ol libérés après 1 heure,
10 - 75 % en poids de 1-diméthylamino-3-(3-méthoxyphényl)-2-méthylpentane-3-ol libérés après 2 heures,
15 - 82 % en poids de 1-diméthylamino-3-(3-méthoxyphényl)-2-méthylpentane-3-ol libérés après 3 heures,
30 - 97 % en poids de 1-diméthylamino-3-(3-méthoxyphényl)-2-méthylpentane-3-ol libérés après 6 heures,
plus de 50 % en poids de 1-diméthylamino-3-(3-méthoxyphényl)-2-méthylpentane-3-ol libérés après 12 heures,
plus de 70 % en poids de 1-diméthylamino-3-(3-méthoxyphényl)-2-méthylpentane-3-ol libérés après 18 heures,
plus de 80 % en poids de 1-diméthylamino-3-(3-méthoxyphényl)-2-méthylpentane-3-ol libérés après 24 heures.

2. Formulation pharmaceutique à libération retardée, qui contient du 1-diméthylamino-3-(3-méthoxyphényl)-2-méthylpentane-3-ol ou un sel pharmaceutiquement acceptable de ce composé dans une matrice à libération retardée de la substance active, la matrice contenant 1 à 80 % en poids d'un ou plusieurs polymères hydrophiles ou hydrophobes comme substance pharmaceutiquement acceptable formant cette matrice, et contient comme substances pharmaceutiquement acceptables formant la matrice, un éther de cellulose et/ou un ester de cellulose ayant une viscosité de 3000 à 150 000 mPa.s, mesurée à 20°C sur une solution aqueuse à 2 % en poids.

3. Composition pharmaceutique suivant l'une des revendications 1 et 2, **caractérisée en ce qu'**elle contient comme substance pharmaceutiquement acceptable formant la matrice, un éther de cellulose et/ou un ester de cellulose, qui présente/présentent une viscosité de 10 000 à 150 000 mPa.s mesurée à 20°C sur une solution à 2 % en poids.

4. Composition pharmaceutique suivant l'une des revendications 1 à 3, **caractérisée en ce qu'**elle contient comme substance pharmaceutiquement acceptable formant la matrice, un éther de cellulose et/ou un ester de cellulose qui présente/présentent une viscosité de 50 000 à 150 000 mPa.s mesurée à 20°C sur une solution à 2 % en poids.

5. Composition pharmaceutique suivant l'une des revendications 1 à 4, **caractérisée en ce qu'**elle contient comme substance pharmaceutiquement acceptable formant la matrice, au moins une substance qui est choisie dans le groupe qui comprend des hydroxypropylméthylcelluloses (HPMC), des hydroxyéthylcelluloses, des hydroxypropylcelluloses (HPC), des méthylcelluloses, des éthylcelluloses et des carboxyméthylcelluloses.

6. Composition pharmaceutique suivant l'une des revendications 1 à 5, **caractérisée en ce qu'**elle contient comme substance pharmaceutiquement acceptable formant la matrice, au moins une substance qui est choisie dans le groupe qui comprend des hydroxypropylméthylcelluloses, des hydroxyéthylcelluloses et des hydroxypropylcelluloses.

7. Composition pharmaceutique suivant l'une des revendications 1 à 6, **caractérisée en ce que** la teneur en substance active devant être libérée de façon retardée se situe entre 0,5 et 85 % en poids et la teneur en substance pharmaceutiquement acceptable formant la matrice se situe entre 8 et 40 % en poids.

8. Composition pharmaceutique suivant l'une des revendications 1 à 7, **caractérisée en ce que** la teneur en substance active devant être libérée de façon retardée se situe entre 3 et 70 % en poids, en particulier entre 8 et 66 % en poids, et la teneur en substance pharmaceutiquement acceptable formant la matrice se situe entre 10 et 35 % en poids, en particulier entre 10 et 30 % en poids.

9. Composition pharmaceutique suivant l'une des revendications 1 à 8, **caractérisée en ce que** le pic du taux de substance active dans le plasma *in vivo* est atteint au bout de 2 à 10 heures, en particulier au bout de 3,5 à 6 heures.

10. Formulation pharmaceutique suivant l'une des revendications 1 à 9, **caractérisée en ce qu'**elle contient du (+)-(2R,3R)-1-diméthylamino-3-(3-méthoxyphényl)-2-méthylpentane-3-ol ou un sel pharmaceutiquement acceptable de ce composé.

11. Comprimé pour l'administration orale deux fois par jour de 1-diméthylamino-3-(3-méthoxyphényl)-2-méthylpentane-3-ol, contenant une formulation pharmaceutique selon l'une des revendications 1 à 10.

12. Utilisation d'une formulation pharmaceutique selon l'une des revendications 1 à 10 ou d'un comprimé selon la revendication 11 pour la préparation d'un médicament destiné au traitement de la répétition du besoin impérieux d'uriner ou de l'incontinence d'urine.

13. Utilisation de 1-diméthylamino-3-(3-méthoxyphényl)-2-méthylpentane-3-ol pour la préparation d'un médicament destiné au traitement de la répétition du besoin impérieux d'uriner ou de l'incontinence d'urine, **caractérisée en ce que** le 1-diméthylamino-3-(3-méthoxyphényl)-2-méthylpentane-3-ol est contenu dans une formulation pharmaceutique selon l'une des revendications 1 à 10.

14. Utilisation d'une formulation pharmaceutique selon l'une des revendications 1 à 10 ou d'un comprimé selon la revendication 11 pour la préparation d'un médicament destiné au traitement de la douleur, en particulier de douleur chronique, viscérale, neuropathique ou aiguë ou d'une douleur inflammatoire.

15. Utilisation de 1-diméthylamino-3-(3-méthoxyphényl)-2-méthylpentane-3-ol pour la préparation d'un médicament destiné au traitement de la douleur, en particulier de douleur chronique, viscérale, neuropathique ou aiguë ou de douleur inflammatoire, **caractérisée en ce que** le 1-diméthylamino-3-(3-méthoxyphényl)-2-méthylpentane-3-ol est contenu dans une formulation pharmaceutique selon l'une des revendications 1 à 10.
